# EUROPEAN PATENT APPLICATION

(11) **EP 0 998 939 A1**
(43) Date of publication of application: **10.05.2000**
(21) Application number: 98307347.9
(22) Date of filing: 09.09.1998
(51) Int. Cl.: A61K 35/78, A61K 31/12, A61K 31/70

(54) **Chalcone plant extracts for use in therapy**

(71) Applicant: THE SCHOOL OF PHARMACY, UNIVERSITY OF LONDON, London WC1N 1AX (GB)
(72) Inventor: Taylor, Elizabeth Jane, Rudgwick, Horsham, W. Sussex, RH12 3JE (GB); Evans, Frederick John, Nr Luton, Beds, LU1 4LU (GB)
(74) Representative: Jones, Helen Marjorie Meredith

(57) **Abstract**

An extract of *Glycyrrhiza* usually *G.glabra* and specific chalcones contained therein, have useful therapeutic properties for the treatment ofpsoriasis. The most active compounds are identified as licochalcone B and echinatin, both of which are more active than isoliquiritigenin, which has adequate performance. The compounds and/or extract are expected to be administered topically to skin affected by psoriasis.

## Description

The present invention relates to the use of plant extracts, specifically chalcones, for use in the treatment of psoriasis. In particular, the invention relates to the use of extracts derived from the *Glycyrrhiza glabra* (liquorice) having particular activity in a psoriasis model in rats.

The prognosis of a patient suffering from psoriasis depends on the age of the individual as well as the extent and severity of the initial outbreak, for example the younger they are greater the severity of the disease. Permanent remissions are rare and present therapy focuses on the management of the disease because no cures exist. It is for this reason and due to the nature of the disease i.e. ranges of severity and the varying forms, that there are so many treatments available which correspondingly also have a wide range in effectiveness and safety.

Corticosteroids penetrate the stratum corneum and bind to steroid receptors in the cytosol of living keratinocytes and function by altering the DNA synthesis and gene transcription, their potency is dependent on the affinity of the receptor for the corticosteroid. Corticosteroids are most useful in treating those with limited psoriasis (<10% body surface), and are most effective when used under occlusive polyethylene covering and applied at night. The toxicity of corticosteroids when most potent includes sever pituitry-adrenal-axis suppression and Cushings syndrome, local side effects includes cutaneous atrophy, rupturing of connective tissue and stretching of the epidermis (Camisa, 1994; BNF, 1997).

Retinoids are naturally or synthetically derived form vitamin A and the effects are similar, they include anti-inflammatory, anti-keratinizing and anti-proliferative activity. Acitretin is a metabolite of etrinate and has generally replaced all other retinoids as it is eight hundred times more potent than etrinate. Acitretin and other retinoids are used for the treatment of severe and resistant or complicated psoriasis. Most patients suffer from dryness and cracking of the lips, other side effects include mild pruritus, paronychia and nose bleeds. It is also a known teratogenic (BNF, 1997).

Methotrexate (MTX) was first used in 1958, and has become the most commonly prescribed systemic anti-metabolite. MTX is a folic acid antagonist and acts by competing for the binding sites on the intracellular enzyme dihydrofolate reductase (DHFR), converting dihydrofolate to tetrahydrofolate. The mechanism of MTX is the inhibition of the (s) phase of the cell cycle and in psoriasis a greater fraction of the cells are in the s-phase. MTX is used in the treatment of acute lymphoblastic leukaemia, meningeal leukaemia, choriocarcinoma, rheumatoid arthritis as well as resistant psoriasis. Most common side effects include bone-marrow depression, ulceration of the mouth and gastro-intestinal disturbances. Other side effects include liver damage, renal failure, tubular necrosis, skin reactions, alopecia, osteoporosis, fertility impairment and teratogenicity (Marindale, 1996). MTX is an effective treatment, but potent side effects can lead to death, therefore it is only used in severe or disabling psoriasis.

Salicylic acid can be used in the treatment of all hyperkeratotic and scaling conditions to enhance the rate of loss of surface scale. Side effects include irritation and occasionally salicylate toxicity.

Dithranol (Anthralin, USA) was first described by Balmano Squire in 1878 as being effective in the treatment of psoriasis. Dithranol, once called Goa powder, is derived from the bark of the Brazilian tree *Andira araoba* Aguiar (Fabaceae). The active compound was the hydroxanthracene 'chrysabin' which on the loss of a methyl becomes dithranol. Dithranol is applied topically to the skin in either the form of an ointment or cream. Treatment schedules often involve coal tar and UV irradiation. Dithranol, however can cause severe irritation in the form of a burning sensation, for this reason it is usual to start with low concentrations and build up. Patients with fair skin are more sensitive than those with dark skin. It is irritant to the eyes and mucous membranes and use should be avoided on skin flexures, genitals and face.

Cyclosporine, a cyclic polypeptide consisting of eleven amino acids, is obtained from the soil fungus *Tolypocladium inflatum* Gams. Cyclosporine A is a unique immunosuppressant because it interferes directly with T-cell function by inhibiting the cytokine cascade (interleukins-I (IL-1) and -2(IL-2) and IL-2 receptor (IL-2R) without myelosuppresive effects. It may also be used in association with PUVA treatment (see below) (Camisa, 1994). Cyclosporine is used in severe and persistent psoriasis only and requires specialist care in hospital. Approximately one third of all patients suffer from nephrotoxicity, other side effects include gastro-intestinal disturbances, hepatoxicity, acne, oedema, neurotoxicity and alopecia. Like many immunosuppresents cylcosporine is associated with an increase incidence of malignancy, lymphoma and skin cancers, as well as the increased risk of infections.

Psoralens are natural compounds from *Psoralea coryfolial* L. (Fabaceae) which interact with sunlight to produce biological effects (psoralens and high-intensity ultraviolet A = PUVA). Xanthotoxin (8-methoxypsoralen, 8-MOP) and the synthetic trioxalen (3,5,8-trimethylpsralen, TMP) are the most commonly used and are given by mouth to sensitise the skin to the effects of irradiation. With activation by light psoralens produce the cross-linking of DNA strands leading to the inhibition of DNA synthesis in the epidermal basal cells. Although this treatment is clean and may produce remissions for several months, short term effects include severe burning and long term hazards include cataract formation, accelerated ageing of the skin and the development of skin cancer. Repeated treatments may increase the risk of skin cancer (BNF, 1997).

Coal tar is a by-product of the processing of coke and gas from bituminous coal, it is a chemical complex of tens of thousands of compounds, described as aromatic hydrocarbons. Coal tar and UVB phototherapy (Goeckerman Regime) suppress the epidermal DNA synthesis; generally coal tar is suitable for most cases but is limited by the unpleasant smell; this treatment may also cause irritation and acne-like eruptions of the skin and should not be applied to inflamed and broken skin. Coal tar is also a recognised carcinogen.

The present invention seeks a treatment for psoriasis which should be efficacious and avoid some of the undesirable side effects of treatments which are in use today or have been proposed.

Flavonoids are naturally occurring compounds present in plants. The minor flavonoids are a category of such compounds which consist of chalcones, aurones, dihydrochalcones, flavanones and dihydroflavonols. These compounds have been extensively reviewed by Bohm, B.A. in "the Flavonoids-Advances in Research", Eds. Harborne, J. and Mabry, T. (1982) Chapman and Hall, pages 313 *et seq.* Chalcones have the general structure 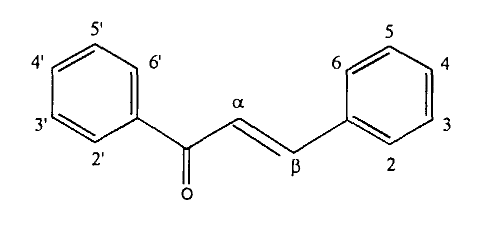 in which there are several hydroxyl or alkoxy substituents in either or both rings.

Of particular interest to the present invention is isoliquiritigenin, the simplest chalcone having one B-ring hydroxyl. This compound has hydroxyl groups at positions 2 and 4 of the A ring and position 4 of the B ring.

Other compounds of particular interest to the present invention are echinatin and licochalcone B, each of which have two B-ring hydroxyls/alkoxy substituents. Echinatin has a hydroxyl substituent at the 4 position of the A ring and hydroxyl at the 4 position of the B ring and methoxy at the 2 position of the B ring. Licochalcone B has hydroxyl at the 4 position in the A ring, two hydroxyl groups at the 4 and 5 positions of the B ring and methoxy at the 2 position of the B ring.

Isoliquiritigenin occurs in *Dahlia* species and *anthorrhoea,* but is most often encountered in members of the *Leguminosae.* Echinatin occurs in *Glycyrrhiza echinata,* whilst licochalcone B occurs in root tissue of *Glycyrrhiza glabra,* along with echinatin and isoliquiritigenin.

Isoliquiritigenin and echinatin have been described to have antitumorogenic properties, as judged by *in vitro* inhibitory activity against phosphorylation of phospholipids promoted by TPA (12-O-tetradecanoylphorbal-13-acetate) in HeLa cells by Shibata, S., Stem Cell (1994) 12, 44-52. Yamamoto S, *et al* in Carcinogenesis (1991) 12 (2), 317-323 also describe antitumour activities of isoliquiritigenin. The assays used are ornithine decarboxylase (ODC) induction and ear edema formation caused by TPA in CD1 mice. Iwata S in Biol.Pharm.Bull. (1995) 18(12), 1710-1713 describe activities of isoliquiritigenin and echinatin on ³²P incorporation into phospholipids of HeLa cells.

Kimura Y in Phytotherapy Research (1993) 7, 335-340 describes the effects of flavonoids isolated from liquorice roots (*Glycyrrhiza inflata* on degranulation in human polymorphonuclear neutrophils. Licochalcone B had an effect whereas isoliquiritigenin had no effect on properties tested, which are associated with inflammatory processes. The results indicate that licochalcone B may be effective in therapeutic limitation of inflammation.

Tawata M. in Eur.J.Pharm.(1992) 212, 87-92, find isoliquiritigenin is tested to have antiplatelet action which they propose is a consequence of its property as an aldose reductase inhibitor. They suggest it may be useful as a possible therapeutic agent in reducing the incidence of diabetes complications. The compound inhibits phosphorylation of some proteins and inhibited formation of 12-(s)-hydroxy-5,8,10-heptadecatrienoic acid, 1 2-hydroxyeicosa tetraenoic acid and thromboxane B2. The level of inhibition of platelet aggregation is determined to be comparable to aspirin in rats.

Extracts of liquorice (*Glycyrrhiza glabra*) have been used as herbal remedies for various inflammatory diseases, though not psoriasis. A compound which had been identified in such abstracts and assumed to be active against inflammation is glycyrrhizic acid.

According to a first aspect of the present invention, there is provided a new use of a chalcone in the manufacture of a composition for use in a method of treatment of psoriasis in a human or animal.

The present inventors believe that chalcones as a class may be proven to have desirable properties in the treatment of psoriasis. The chalcones should generally be extracted from a natural source, which is preferably a plant known to be a good source of chalcones, for instance selected from those described by Bohm, *op. cit.* The chalcones used to manufacture the composition may often be used as mixtures of naturally co-occurring chalcones, often also with other co-occurring compounds which are extracted along with the chalcones from the plant source. The chalcone may be synthetic rather than extracted form a natural source, or may be a derivative of a natural compound.

The or each chalcone should preferably be of the general formula I: 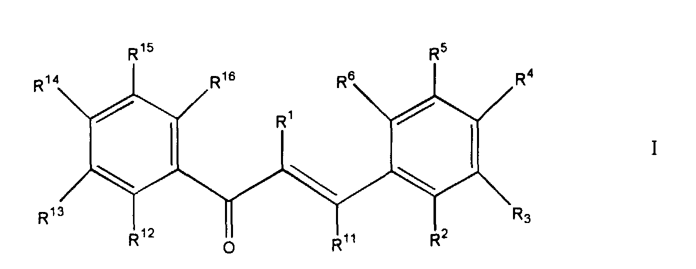 in which the groups R² to R⁶ and R¹² to R¹⁶ may be the same or different and each represents hydrogen, C₁₋₂₄ alkyl, C₁₋₂₄ alkenyl, or OR¹⁰ where R¹⁰ is hydrogen, C₁₋₂₄ alkyl, C₁₋₂₄ alkenyl, a glycoside or oligosaccharide joined by O-glycosidic bond, C₁₋₂₄-alkanoyl, or C₁₋₂₄-alkenoyl, and wherein adjacent R groups in the A ring or the B ring can be joined to make five or six membered rings with the carbon atoms to which they are attached, optionally including an etheric or lactam linkage, and optionally including unsaturation;
R¹ and R¹¹ are each selected from hydrogen, hydroxyl, C₁₋₆ alkyl, C₁₋₆ alkoxy, C₁₋₆ alkenyl and C₁₋₆ alkenoxy or may be joined to form a ring, optionally a fused ring with the chalcone B ring, which ring has five or six carbon atoms and may include additional ethylenic unsaturation,
provided that:
   i) at least one of R¹² and R¹⁴ is OR¹⁰; and
   ii) where either R¹² or R¹⁴ is selected from hydrogen, alkyl or alkenyl, both R² and R⁴ is OR¹⁰.

Alkenyl substituents are generally prenyl , isoprenyl or oligomeric versions of these groups such as geranyl.

Alkyl and alkeny groups generally containd 1 to 6 carbon atoms and may be straight or branched.

Glycosidic substituents are generally glucose but may be any other sugar.

Where two adjacent R groups are joined to form a five or six membered ring with the carbon atoms to which they are attached, they generally represent -O-CH₂-O-, -O-(CH₂)-₂, -O-CH=CH-, -O-C(CH₃)₂-CH=CH, -O-C(CH₃)₂-CHOH-CH₂-, -O-CO-(CH₂)-₂. Sometimes even more complex structures may be formed with three fused rings, formed by three adjacent groups R²⁻⁶ or R¹²⁻¹⁶ together with the carbon atoms to which they are attached.

One example of a chalcone which has some useful properties for the treatment of psoriasis is isoliquiritigenin. This compound has the formula I, in which R¹², R¹⁴ and R⁴ are each hydroxyl and each of the other groups R¹ to R⁶ and R¹¹ to R¹⁶ represents hydrogen.

Preferably the chalcone is of a more limited general formula in which at least two of the groups on the B ring R² to R⁶ represents OR¹⁰. According to a preferred aspect of the invention therefore the chalcone has the general formula II 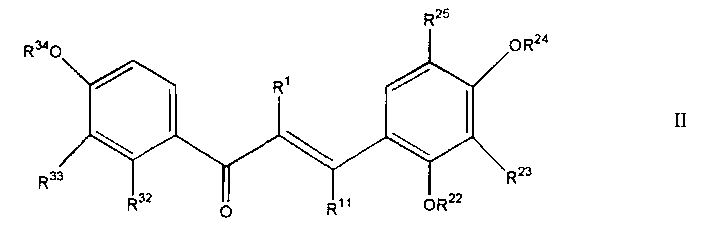 in which R²² represents hydrogen or C₁₋₆ alkyl, preferably methyl;
R²³ represents hydrogen, hydroxyl, C₁₋₆-alkyl, C₁₋₆-alkenyl, C₁₋₆ alkoxy, C₁₋₆ alkenyloxy, C₁₋₆ alkanoyloxy, C₁₋₆ alkenyoyloxy, O-glycoside, preferably hydrogen or hydroxyl;
R²⁴ is hydrogen or a C₁₋₆ alkyl group, preferably hydrogen;
R²⁵ is hydrogen, C₁₋₆ alkyl or C₁₋₆ alkenyl, preferably hydrogen;
or R²³ and OR²⁴ are joined to form -O(CH₂n)-O-, in which n is 1 or 2;
R³² is hydrogen, hydroxyl, C₁₋₆ alkyl, C₁₋₆ alkenyl, C₁₋₆ alkoxy, C₁₋₆ alkenyloxy, O-glycoside or O-oligosaccharide;
R³³ is hydrogen or C₁₋₆ alkyl or C₁₋₆ alkenyl;
R³⁴ is hydrogen, C₁₋₆ alkyl, a glycoside or oligosaccharide; or
R³³ and R³⁴ may together form a straight or branched alkylene or alkenylene whereby a five or six membered heterocyclic ring is formed; and
R¹¹ and R¹ are as defined above.

Preferably R¹, R¹¹, R³², R³³ and R³⁴ are each hydrogen. Preferably R²⁴ is also hydrogen, whilst R²² is hydrogen or, preferably, methyl. R²³ is preferably either hydroxyl or hydrogen. R²⁵ is preferably hydrogen, lower alkyl or lower alkenyl.

Most preferably the compound of the formula II is echinatin (R¹, R¹¹, R³², R³³, R³⁴, R²⁵, R²⁴, R²³ are each hydrogen and R²² is methyl) or is licochalcone B (as echinatin except that R²³ is hydroxyl).

In the composition containing the chalcone(s) there is preferably a pharmaceutical excipient selected so as to be suitable for the proposed route of administration. In the invention it is proposed that the chalcone(s) are administered topically, in the form of a cream or lotion. Accordingly the pharmaceutical excipient provides a suitable composition for application by such means. The vehicle may include water and/or organic solvent

It is preferred that the chalcones be present in a total concentration of at least 0.01% in the composition, preferably in an amount of at least 0.1%, for instance up to 10% or, preferably, up to 5% by weight.

The chalcones used in the invention, especially where these contain echinatin, licochalcone B and/or isoliquiritigenin, may be co-extracted, for instance from plants of the *Glycyrrhiza* genus, most preferably *Glycyrrhiza glabra.* The method of extraction preferably involves a solvent extraction method using solvents of sequentially increasing polarity.

According to a further aspect of the invention, there is provided a new use of an extract of *Glycyrrhiza* in the manufacture of a composition for use in a method of treatment of psoriasis.

The *Glycyrrhiza* extract is preferably obtained using a solvent extraction process including several steps using sequentially increasingly polar solvents. Chalcones are not extracted by relatively non polar solvents, for instance having dielectric constant less than 5. It is preferred therefore that the *Glycyrrhiza* source has previously been extracted using a non polar solvent, for instance having a dielectric constant up to 5, for instance selected from C₆ or C₇ alkanes, carbon tetrachloride, toluene, trichloroethylene, diethylether and chloroform. The chalcones are then preferably extracted from the remaining material using a relatively polar solvent, having a dielectric constant higher than that of the first solvent and preferably more than 5. For some purposes it may be satisfactory to co-extract other components soluble in highly polar solvents, and thus to use a very polar solvent such as an alcohol, acetone or, possibly, water. Often it may be desired that co-occurring compounds which are soluble in such highly polar solvents should not be co-extracted. It is therefore preferable to use a solvent having a dielectric constant in the range 5 to 10, such as ethyl acetate or dichloromethane. The solvent containing the dissolved extract is preferably worked up to recover the desired extract in solid or more concentrated solution or suspension form.

The present inventors have established that a good source of the chalcones having particular activity in a psoriasis model and in other assays believed to correlate with psoriasis treatment, echinatin and licochalcone B, is *Glycyrrhiza glabra.* A particularly preferred embodiment of the second aspect of the invention therefore utilises a solvent extract of *glycyrrhiza glabra.* Other *Glycyrrhiza* species which may be suitable sources of active chalcones are *G. echinata, G. glandulifera, G. hirsuta, G. brachycarpa, G. uralensis, G. pallidflora, G. korshinski, G. inflata, G. lepidota and G. radix.*

The present invention is illustrated further in the accompanying examples

### Example 1. Isolation of Compounds

### 1.1 Extractive Process (A) (Figure 1)

A soxhlet apparatus was used to continuously extract compounds from the plant material. Soxhlet extraction is a form of continuous percolation with fresh solvent, and uses special glassware. The plant material is separated from the extract by encasing in a paper 'thimble' beneath dripping condensed solvent. When full, the solvent in the thimble siphons off to the main vessel containing the extract and the process continues. The advantage being that fresh solvent continuously extracts the plant material more efficiently with minimum solvent. Originally, as the active constituents were unknown, a non-polar solvent (hexane) was used to exhaustively extract the plant material, followed by a series of more polar solvents (chloroform, ethyl acetate, ethanol and water), until several extracts were obtained of increasing solute polarity. Approximately 100g was extracted on each occasion and this was repeated several times and the extracts grouped together according to their constituents.

The five extracts were subjected to the screening programme, inhibition of chemically induced erythema on the mouse ear, using 12-O-tetradecanoylphorbol-13-acetate, this technique was also used for the bioassay guided fractionation. The ethyl acetate fraction was most active, and future extraction could be done far more efficiently using a selective solvent extraction process.

### Fractionation Using Chromatography

Chromatography is often defined as the separation of solutes in a mixture according to their affinity for a stationary phase, through which a mobile phase moves. It is used for analysis of small samples as well as on a large scale for preparation of fractions.

### 1.2 Column Chromatography (Silica) (B)

The ethyl acetate extract was placed on top of the adsorbent (silica gel) and eluted through a column with progressively more polar solvents. Approximately lg was placed onto a column with dimensions of 20cm x 1.5cm. Chloroform was used as the non-polar solvent and polarity was gently increased by adding methanol in the following percentages, 2, 5, 10, 20% with a final wash of 50% methanol/chloroform. The quantity of solvent added at each concentration was determined by thin layer chromatography (TLC) and whether the extract had been exhaustively extracted. The active fractions containing isoliquiritigenin, licochalcone B and echinatin, when collected in 100ml quantities, were numbers 4 and 5 for isoliquiritigenin and numbers 8, 9 and 10 for liochalcone B and echinatin, this was determined by using TLC

### 1.3 Column Chromatography (Polyamide) - Separation of Samples 8, 9 and 10 (C )

A column containing polyamide gel (mesh200) was set up with dimensions of 5cm x 1cm. A more complicated solvent system was used to elute the column,
Chloroform (20): Methanol (10): Isopropanol (5): Acetone (1)

The methanol was increased from 10ml in 80ml quantities to 15, 20, 25ml to a final wash with methanol only. Approximately 0.1 g was loaded on to the column each time and 5ml fractions collected.

Similar fractions were grouped together. At this stage two dominant compounds were visible using polyamide TLC

### 1.4 Preparative TLC using Polyamide Plates (D.1)

Preparative TLC is used in the same way as analytical TLC except the layer is thicker and takes a higher loading of extract. The extract is spotted continuously along the base-line and the plate developed in the usual way ( Chloroform 20:Methanol 10: Isopropanol 5 and Acetone 1). Visualisation was by ultra-violet light. The zones containing the required fractions were scraped off and eluted with a suitable solvent. These fractions were then concentrated down separately to give pure compounds.

(D2.) The same preparative TLC system was used to purify isoliquiritigenin from fractions 4 and 5 from the silica gel column (B).

### Example 2. Identification of Compounds

Identification was achieved by spectroscopic techniques including UV analysis, mass spectrometry and one and two dimensional NMR. Initial identification of the group of compounds (i.e. flavanoids > chalcones), could be done through spraying TLC plates with special reagents and observing colour changes both in normal daylight and under UV (254nm and 365nm). However, identification of the individual compounds was done using the techniques already mentioned and comparing to data already in the published literature.

### 2.1 UV Analysis

The visible and ultraviolet spectra of organic compounds are associated with transitions between electronic energy levels. The transitions are generally between a bonding or lone-pair orbital and an unfilled non-bonding or anti-bonding orbital. The wavelength of the absorption is then a measure of the separation of the energy levels of the orbitals concerned. At above 200nm, excitation energy of electrons from p and d-orbitals and π-conjugated systems gives rise to readily measured and informative spectra. The spectra is the information used to facilitate the identification of the compounds.

### 2.2 Mass Spectrometry (MS)

The mass spectrometer is designed to perform three functions: vaporize compounds of widely varying volatility; produce ions from the resulting gas-phase molecules; and to separate ions according to their mass-to-charged ions ratios (*m/z*) and subsequently detect and record them. The mass spectrometer is a device for the production and weighing of ions. The mass-to-charged ion ratios for the three compounds are as follows:

| | |
|---|---|
| isoliquiritigenin | - 256*m/z* |
| licochalcone B | - 286*m/z* |
| echinatin | - 270*m/z* |

### 2.3 Nuclear Magnetic Resonance (NMR)

NMR spectroscopy essentially provides a means of determining the structure of an organic compound by measuring the magnetic moment ofits hydrogen atoms. In most compounds, hydrogen atoms are attached to different groups (as -CH₂, -CH₃, CHO, NH₂, -CHOH etc.) and the NMR spectrum provides a record of the number of hydrogen atoms in these different situations. It cannot, however give any direct information on the nature of the carbon skeleton of the molecule; this information in the first instance must be obtained by application of other special techniques, for example carbon-13.

### Example 3. Topical Chemical Erythema

Ref. Williamson E.M. and Evans F. (1981), Inhibition of erythema induced by proinflammatory esters of 12-deoxyphorbol. Acta Pharmacol. Toxicol.,48, 47-52

This biological test involves the determination of chemically induced erythema on the mouse ear and was used for the bioassay guided fractionation.
1. Female CD1 mice, weighing approximately 20g were used.
2. Test plant extracts as produced in Example 1 were applied externally to the right ear of each mouse, at predetermined concentrations in 5µl quantities, 20 minutes prior to the application of the irritant compound 12-O-tetradecanoylphorbal-13-acetate (TPA)(0.1µg/5µl).
3. A negative response was indicated by redness and inflammation of the ear.
4. Observations were recorded at 2,3,4,6,7, and 24 hours after the application of TPA. Maximum inflammation caused by TPA alone occurs at 6 hours after the application of TPA.

### Results

Erythema of the mouse ear is the simplest experiment to perform as mice have translucent ears in which reddening is easily visible. Quantification is subjective, however the erythema can be described as pronounced, slight or absent.
Isoliquiritigenin ED50 (dose required to inhibit erythema in at least 50% of the animals) 20-50,µg
Licochalcone B ED50 5-10µg
Echinatin ED50 5-10µg

Both licochalcone B and echinatin are the most effective in inhibiting the TPA induced erythema. Although all three compounds are chalcones, licochalcone B and echinatin are classified as retrochalcones, they lack the hydroxy-groups at C-2' and C-6' in the A-ring and possesses the resorcinol type B-ring.

### Example 4 Inhibition of Blood Platelet Aggregation

Blood platelets release inflammatory mediators in response to an external stimulus, which affect the proliferation of the cells, which is an important part of the disease psoriasis.
1. Male human blood was collected via venipucture of the forearm directly in to anticoagulant buffer, from healthy donors who had denied any medication for two weeks, and centrifuged at 1000rpm for 20 minutes at room temperature.
2. The supernatant was employed as platelet rich plasma (PRP). Platelet poor plasma (PPP) was obtained by centrifuging 1ml of PRP at 11,000 rpm for 10 minutes.
3. Inhibition of platelet aggregation was tested by adding 2.5µl of a known concentration (containing 10µg or 50µg) of the test compound to a 500µl sample ofPRP for one minute. An aggregation agent was then added as a solution to the sample in a 2.5µl quantity (containing TPA 0.5,µg or ADP 6.4 µg). Aggregation was measured using a dual channel aggregometer .
4. Aggregation was taken as the % change in light transmission compared to the maximum change produced by TPA or ADP plus the control solution. PRP was used as a standard for zero light transmission and PP for 100% light transmission.

### Results

**Table 1.**

| Percentage Inhibition of TPA and ADP Induced Aggregation By The Three Chalcones at 50 and 10µg. | | | | |
|---|---|---|---|---|
| Compounds | Conc. 50µg | | Conc. 10µg | |
| | TPA | ADP | TPA | ADP |
| Isoliquiritigenin | 13 | 26 | 3 | 20 |
| Echinatin | 6 | 14 | NT | 13 |
| Licochalcone B | 13 | 15 | 4 | NT |
| NT = not tested | | | | |

The purpose of this experiment was to observe the effects on platelet aggregation and the possible modes of action i.e., which biological pathway the compunds maybe inhibiting.

When platelets interact with substances such as ADP aggregation may occur, at first this is reversible but a second aggregation will occur whereby pharmacologically active substances (e.g. ADP, serotonin and eicosanoids) are released and the process becomes irreversible. ADP induces the release of arachidonic acid from the membrane phospholipids resulting in the synthesis of prostaglandins and thromboxanes A2.

TPA is known to cause irreversible platelet aggregation at 0.2µM. TPA induced aggregation does not occur via the arachidonic pathway. The protein kinase C enzyme is present in high amounts in platelets and since this is the major receptor of TPA it is thought the direct aggregatory response could be mediated through this enzyme.

The objective was to observe the different responses of the compounds to two different pathways of induced platelet aggregation, which could give an indication to the compounds mechanism of action. Isoliquiritigenin was the most active in inhibiting blood platelet aggregation, especially against the ADP induced aggregation. All three compounds showed greater inhibition against ADP aggregation and at 10ug had little effect against TPA induced aggregation.

### Example 5. UV-B Induced Erythema In Rat

Physically induced erythema is carried by UV-B light. Rat UV-B induced dermatitis is characterised by a brownish red lesion with scab formation. Histologically, microvascular dilation, intraepidermal accumulation ofleukocytes and hyperproliferation of epidermal cells are all very similar to the symptoms of psoriasis in man (Nakaguma,H., Int. J.ofExp.Path, 76:65-73, 1995)
1. Hair on the dorsal skin of male Wistar rats (180g) was clipped and shaved, whilst the rats were under anaesthesia, 24 hours prior to the experiment.
2. The rats were covered by a black plastic sheeting, with two rectangular holes (about 1cm²) cut out, thus allowing two exposure sites on the dorsal skin whilst protecting the rest of the animal.
3. The rats were then exposed to UV-B light (302nm) at a distance of 10cm for six minutes and thirty seconds. The total W-B exposure was 2.4J/cm² as measured using a UV-X radiometer.
4. The test compound was then placed onto one of the exposed areas and an unexposed area at a concentration of 100µg/5µl, in a suitable vehicle solution.
5. Application of the test compound was repeated for five days.
6. Histological post-mortem sections were taken on the fifth day, and stained using haematoxylin and eosin. The number ofepidermal cell layers and the epidermal thickness was counted and measured at random. Five animals were used per test group.

### Results

**Table 2.**

| Percentage increase/decrease in epidermal proliferation and thickening as compared to the control (no treatment). Percentage inhibition of epidermal proliferation and thickening is shown in brackets. | | | | | | |
|---|---|---|---|---|---|---|
| Compound | UV Exposure | | UV Exposure + Active Compound | | Active Compound Only | |
| | Thickness | No. of cells | Thickness | No. of cells | Thickness | No. of cells |
| Isoliquiritig- | 155 | 182 | 116 | 119 | 102 | 103 |
| enin | | | (71) | (77) | | |
| Licochalcone | 240 | 269 | 135 | 129 | 96 | 103 |
| B | | | (75) | (83) | | |

This model clearly shows the anti-erythema activity of both isoliquiritigenin and licochalcone B. The percentage of inhibition is between 70 and 75 % for both compounds. This is probably some of the most significant results of the research as UV-B induced dermatitis in rats is so similar to psoriasis, expressing very similar symptoms.

### Example 6 Cell Culture 3[H] Thymidine Assay

Cells used were swiss 3T3 mouse fibroblast cells
1. 6 x 24 well plates were required for each experiment.
2. Cells were removed from the standard tissue culture flask using a 1% solution of EDTA and resuspended in 10 ml of media and pipetted off into a falcon tube.
3. The cells were then spun for 5 minutes at about 2,000rpm.
4. The existing media was removed, and the cells again resuspended in 10ml of 10% foetal calf serum (FCS) and vortexed.
5. The 10ml of suspension was then added to 140ml of media and mixed in a sterile jar. 1ml of the media cell suspension was then placed into each well and the plates incubated until confluent, three days .

### Assay

1. [PCl]Cells were dosed with the test compounds (as specified below) 2 days prior to the addition of 3 [H]thymidine. The cells could only be dosed with a 0.5% solution, 5µl test solution in 1ml cellsuspension i.e. 5µl in 1ml, (TPA - 0.5µM).
2. 0.5µl of 3[H] thymidine was added per well, therefore 72µl was added to 144µl of media (1%F.C.S.) to dose the corresponding six plates.
3. 100µl of the media was taken to measure the measure the radioactive dose 37Bq=lmCi, and added to 4ml of scintillation fluid.
4. The radioactive media was then transferred to sterile specimen tubes and dosed with the test compounds before adding to the cells.
5. After four hours ofbeing pulsed with radiolabelled thymidine (0.5µCi/ml) the media was removed and washed down the sink.
6. The cells were washed twice with ice cold phosphate buffered saline (PBS), 0.5ml per well, and incubated for 30 min using ice cold 5% trichloroacetic acid (TCA) ( 1ml) per well at room temperature.
8. The TCA was then removed and another 1ml of 5% TCA was added for 5 minutes. After the TCA was removed and 0.3NaOH and 0.1% sodium dodecyl sulphate added to each well for 30 minutes (1ml/well).
9. The contents of each well was added to scintillation vials containing 4ml of scintillation fluid and each sample counted for four minutes using a scintillation counter.
Three different assays were carried out using this protocol;
6.1. Cytotoxicity testing of the compounds. After 48 hours of incubation and the cells were confluent a predetermined quantity of the test compounds was added to the cells.
6.2. Inhibition of TPA induced proliferation. After 48 hours a preditermined quantity concentration of the test compounds was added to the cells as well as 0.5um of TPA. The objective of this experiment was to see if the compounds could successively inhibit the proliferation of cells especially at doses whereby they were not cytotoxic to normal cell growth.
6.3. Inhibition of TPA induced proliferation. After 48 hours a predetermined quantity concentration of the test compounds was added to the cells for 24 hours, at 72 hours the media was changed again to contain both test compound and 0.5µM of the TPA. The objective of this experiment was to observe whether the prior incubation with the test compound had any effect on the TPA induced proliferation of the cells.

### Results

**Table 3.**

| Percentage Inhibition of 3T3 Swiss Fibroblast Growth | | | | |
|---|---|---|---|---|
| Compound | Concentration µg | Example 6.1 | Example 6.2 | Example 6.3 |
| Isoliquiritigenin | 20 | 54 | 29 | 61 |
| | 10 | 32 | 28 | 47 |
| | 1 | 32 | 4 | 50 |
| | 0.1 | 18 | +4 | 39 |
| | 0.01 | 20 | 6 | 31 |
| Licochalcone B | 20 | 86 | 73 | 85 |
| | 10 | 55 | 32 | 69 |
| | 1 | 41 | 31 | 22 |
| | 0.1 | 13 | 17 | 37 |
| | 0.01 | 16 | +26 | 2 |
| Echinatin | 20 | 99 | 99 | 96 |
| | 10 | 94 | 98 | 96 |
| | 1 | 85 | 42 | 65 |
| | 0.01 | 28 | 15 | 39 |
| | 0.01 | 3 | +5 | 30 |

Although work has previously been done using HeLa and isoliquiritigenin and echinatin (*op.cit*), none has been done using 3T3 cells or on licochalcone B. From these studies it is evident that echinatin is the most active, on normal cell growth it was capable of inhibition up to 82% at 1µg in these experiments, however further studies would be needed to establish the exact EC50 (between 0.1 and 1µg). Interestingly the compounds were able to inhibit TPA induced proliferation to the same extent asthey inhibit normal cell growth, however this was not the case when the compounds were incubated with the cells prior to the addition of TPA (with the exception of licochalcone B). These results prove that the compounds are able to inhibit cell growth but more importantly able to inhibit TPA induced cell proliferation.

### References

British National Formulary (BNF), No. 34, Sep., 1997.

British Medical Association and Royal Pharmaceutical Society of Great Britain

Martindale, The Extra Pharmacopeia, 31st Edition, 1996

The Royal Pharmaceutical Society

Psoriasis, Carnisa, C., 1994, Blackwell Scientific Press

## Claims

1. Use of a chalcone in the manufacture ofa composition for use in a method of treatment of psoriasis in a human or animal.

2. Use according to claim 1 in which the chalcone is an extracted from a natural source, preferably from a *Glycyrrhiza* species.

3. Use according to claim 1 or claim 2 in which the chalcone has the general formula I: 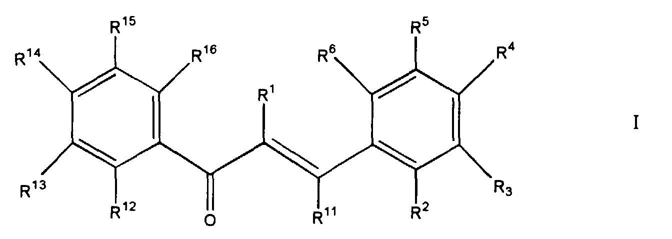 in which the groups R² to R⁶ and R¹² to R¹⁶ may be the same or different and each represents hydrogen, C₁₋₂₄ alkyl, C₁₋₂₄ alkenyl, or OR¹⁰ where R¹⁰ is hydrogen, C₁₋₂₄ alkyl, C₁₋₂₄ alkenyl, a glycoside or oligosaccharide joined by O-glycosidic bond, C₁₋₂₄-alkanoyl, or C₁₋₂₄-alkenoyl, and wherein adjacent R groups in the A ring or the B ring can be joined to make five or six membered rings with the carbon atoms to which they are attached, optionally including an etheric or lactam linkage, and optionally including unsaturation;
R¹ and R¹¹ are each selected from hydrogen, hydroxyl, C₁₋₆ alkyl, C₁₋₆ alkoxy, C₁₋₆ alkenyl and C₁₋₆ alkenoxy or may be joined to form a ring, optionally a ring fused with the chalcone B ring, which ring has five or six carbon atoms and may include additional (to the B ring) ethylenic unsaturation
provided that:
i) at least one of R¹² and R¹⁴ is OR¹⁰; and
ii) where either R¹² or R¹⁴ is selected from hydrogen, alkyl or alkenyl, both R² and R⁴ is OR¹⁰.

4. Use according to claim 3 in which, where two adjacent R groups are joined to form a five or six membered ring with the carbon atoms to which they are attached, they generally represent -O-CH₂-O-, -O-(CH₂)-₂, -O-CH=CH-, -O-C(CH₃)₂-CH=CH, -O-C(CH₃)₂-CHOH-CH₂-, -O-CO-(CH₂)-₂.

5. Use according to claim 3 in which
R¹ = R² = R³ = R⁵ = R⁶ = R¹¹ = R¹³ = R¹⁵ = R¹⁶ = H and R⁴ = R¹² = R¹⁴ = OH.

6. Use according to claim 1 or claim 2 in which the chalcone has the general formula II 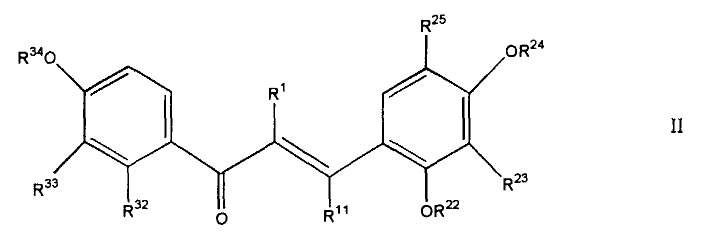 in which R¹ and R¹¹ are each selected from hydrogen, hydroxyl, C₁₋₆ alkyl, C₁₋₆ alkoxy, C₁₋₆ alkenoxy or may be joined to form a ring, optionally a fused ring with the chalcone B ring, which ring has five or six carbon atoms and may include additional ethylenic unsaturation;
R²² represents hydrogen or C₁₋₆ alkyl;
R²³ represents hydrogen, hydroxyl, C₁₋₆-alkyl, C₁₋₆-alkenyl, C₁₋₆ alkoxy, C₁₋₆ alkenyloxy, C₁₋₆ alkanoyloxy, C₁₋₆ alkenyoyloxy, O-glycoside;
R²⁴ is hydrogen or a C₁₋₆ alkyl group;
R²⁵ is hydrogen, C₁₋₆ alkyl or C₁₋₆ alkenyl;
or R²³ and OR²⁴ are joined to form -O(CH₂)n-O-, in which n is 1 or 2;
R³² is hydrogen, hydroxyl, C₁₋₆ alkyl, C₁₋₆ alkenyl, C₁₋₆ alkoxy, C₁₋₆ alkenyloxy, O-glycoside or O-oligosaccharide;
R³³ is hydrogen or C₁₋₆ alkyl or C₁₋₆ alkenyl; and
R³⁴ is hydrogen, C₁₋₆ alkyl, a glycoside or oligosaccharide; or
R³³ and R³⁴ may together form a straight or branched alkylene or alkenylene whereby a five or six membered heterocyclic ring is formed;

7. Use according to claim 6 in which
R²² is methyl;
R²³ is hydrogen or hydroxyl; and
R¹, R¹¹, R²⁴, R²⁵, R³³ and R³⁴ are each hydrogen.

8. Use according to claim 7 in which the chalcone is licochalcone B or echinatin.

9. Use of an extract of *Glycyrrhiza* in the manufacture of a composition for use in a method of treatment of psoriasis.

10. Use according claim 9 in which the extract is obtainable by a multistep process including
a) a first step in which *Glycyrrhiza* is extracted with a polar solvent having a dielectric constant of less than 5;
b) a second step in which the solids remaining after step a are contacted with a relatively polar solvent, having a dielectric constant higher than that of the first solvent and preferably more than 5; and
c) a further step of recovering the solutes in the solution obtained in step b) in solid or more concentrated solution or dispersion form.

11. Use according to claim 9 or claim 10 in which the *Glycyrrhiza* is *G.glabra.*

12. Use according to any preceding claim in which the composition is for topical administration to skin affected by psoriasis.
